# EUROPEAN PATENT SPECIFICATION

(11) **EP 4 426 283 B1**
(45) Date of publication and mention of the grant of the patent: **30.09.2026**
(21) Application number: 22891065.9
(22) Date of filing: 03.11.2022
(51) Int. Cl.: A61K 31/225, A61P 3/02, A61P 43/00, A23L 33/10

(54) **KETONE PRECURSORS AND METHODS THEREFOR**
KETONVORLÄUFER UND VERFAHREN DAFÜR
PRÉCURSEURS DE CÉTONE ET PROCÉDÉS CORRESPONDANTS

(30) Priority: 04.11.2021 US 202163275858 P
(43) Date of publication of application: 11.09.2024
(73) Proprietor: VDF Futureceuticals, Inc., Momence, Illinois 60954 (US)
(72) Inventor: ANDRYIANAU, Gleb, 02-676 Warsaw (PL); PIETRZKOWSKI, Zbigniew, Momence, Illinois 60954 (US)
(74) Representative: Hutter, Anton
(86) International application number: PCT/US2022/079256
(87) International publication number: WO 2023/081786

(56) References cited:
- EP-A2- 2 283 834
- WO-A1-01/01960
- WO-A1-2020/186154
- CN-A- 111 840 321
- US-A- 4 701 443
- US-A1- 2016 168 041
- US-A1- 2018 195 096
- US-A1- 2019 014 798
- US-A1- 2020 113 220

## Description

### Field of the Invention

The field of the invention is compositions and methods for delivering a precursor of (R)-3-hydroxybutyrate to a mammal, and especially as it relates to compounds that are formulated as a precursor that is formed from a metabolically-relevant (di)carboxylic acid and 1,3-butanediol.

### Background of the Invention

The background description includes information that may be useful in understanding the present invention. It is not an admission that any of the information provided herein is prior art or relevant to the presently claimed invention, or that any publication specifically or implicitly referenced is prior art.

Nutritional, or therapeutic, ketosis is the physiological state of elevated blood ketone body levels (typically above 0.5 mmol/L) resulting from ketogenic diets, calorie restriction, therapeutic fasting, and/or supplementation with ketogenic precursors. Ketone bodies represent alternative energy substrates for both peripheral tissues and the central nervous system. The two most abundant and physiologically significant ketone bodies are acetoacetate and (*R*)-3-hydroxybutyrate (also referred to as beta-hydroxybutyrate), while the third ketone body, acetone, is produced as a byproduct that the lungs breathe off. The body produces ketone bodies during nutritional or therapeutic ketosis, and the metabolism of ketone bodies is associated with anticonvulsant effects, enhanced brain metabolism, neuroprotective, muscle sparing properties, and improvement in cognitive and physical performance. Science-based improvements in efficiency of cellular metabolism, managed through ketone supplementation, could have beneficial impacts on physical, cognitive health, psychological health, warfighter resilience, and a long-term impact on health with respect to the common avoidable diseases such as obesity, neurodegenerative diseases, diabetes, and cancer, alleviate fatigue, and may also provide anti-aging effect, reduce aging of skin and other tissues and organs, and may have modulatory effect on immunity and inflammation.

During periods of carbohydrate deprivation, the body utilizes energy obtained from the metabolism of fats. During fat metabolism, fats are converted to acetoacetate and 3-hydroxybutyric acid, which are known as ketone bodies, and large quantities of these substances accumulate in the blood. This condition, which is known as ketosis, commonly occurs during starvation. When blood ketone body concentrations are elevated to levels found in prolonged starvation, they provide the major source of energy for the brain. In addition to being a unique, high energy metabolic substrate, (*R*)-3-hydroxybutyrate has a variety of potential therapeutic applications. For example, cardiac efficiency and brain metabolic efficiency are increased and the effects of neurodegenerative disorders, such as Alzheimer's and Parkinson's diseases are reduced. Moreover, (*R*)-3-hydroxybutyrate can serve as an alternative physiologic energy source.

While (*R*)-3-hydroxybutyrate could be administered directly, such direct administration is not practical. For example, (*R*)-3-hydroxybutyrate is relatively expensive, tastes bitter, and could lead to undesirable side effects due to significant acidosis following rapid absorption from the gastrointestinal tract. Moreover, where hydroxybutyrate is provided as a salt, sodium levels will readily become unacceptable where higher doses of hydroxybutyrate are consumed. In an attempt to avoid ingestion of large quantities of sodium or other cations, various (*R*)-3-hydroxybutyrate derivatives were tested to ultimately deliver (*R*)-3-hydroxybutyrate as a metabolite. However, synthesis of all or almost all of these derivatives require expensive precursors and/or have undesirable S-isomeric byproducts. Among other examples, known (*R*)-3-hydroxybutyrate derivatives include 3-hydroxybutyl-(*R*)-3-hydroxybutyrate (US 2019/0014798) D-β-hydroxybutyryl-acetoacetate ester, acetoacetyl ester of oligomeric (R)-3-hydroxybutyric acid (EP2283834A2), beta hydroxybutyrate citrate ester (WO2020186154A1) and mixed (*R*)-3-hydroxybutyrate oligomers (US 2018/0195096). In still further examples, as discussed in US 2020/0113220, a monoester can be formed between (*R*)-3-hydroxybutyrate and 1,3-butanediol. However, such monoester once more requires stereochemically pure reagents, adding to product cost and potential issues with enantiomeric purity.

In yet other approaches that employ 1,3-butanediol as a metabolic precursor to (*R*)-3-hydroxybutyrate, an exogenous ketone diester, bishexanoyl-(*R*)-1,3-butanediol (BH-BD) has been used as a dietary source for ketone precursor delivery. Here the diester is formed from one molecule of (*R*)-1,3-butanediol with two molecules of hexanoic acid to produce BH-BD. The BH-BD diester is hydrolyzed upon ingestion and the resulting (*R*)-1,3-butanediol is converted in the liver in a non-classical step to (*R*)-3-hydroxybutyrate while the remaining hexanoic acid molecules are converted in the liver in a classical (beta oxidative) step to (*R*)-3-hydroxybutyrate. Further exemplary diester compounds are described in US 2019/0248730. Similarly, US 2021/0186914 teaches a *R,S*-1,3-butanediol acetoacetate diester that was used for treatment of cachexia. Here, the diester is formed from one molecule 1,3-butanediol and two molecules acetoacetic acid.

While at least some of such compounds and compositions tend to be less expensive to manufacture and/or will not provide excess quantities of sodium or other cations when ingested in significant quantities, various disadvantages nevertheless remain. Among other things, palatability may be poor, and at least some of these compounds will have undesirably low solubility in aqueous media. Still further, many of these compounds have low chemical stability and non-enzymatic hydrolysis of these compounds will result in racemic 3-hydroxybutyrate, of which one stereoisomeric form is not significantly metabolized into energy.

Thus, even though various compositions and methods for (*R*)-3-hydroxybutyrate or precursors thereof are known in the art, all or almost all of them suffer from several drawbacks. Therefore, there remains a need for improved compounds, compositions, and methods for *(R)-*3-hydroxybutyrate and precursors thereof.

### Summary of The Invention

The inventive subject matter is directed to various compounds, compositions, and methods to provide 3-hydroxybutyrate, and particularly (*R*)-3-hydroxybutyrate, to a mammal via a compound that delivers a precursor from which (*R*)-3-hydroxybutyrate is generated *in vivo* and that further generates a metabolically relevant second compound.

In preferred aspects, upon oral administration of contemplated compounds, *in vivo* generation of (*R*)-3-hydroxybutyrate proceeds via enzymatic hydrolysis of contemplated compounds to form (*R*)-1,3-butanediol, which is further enzymatically oxidized to (*R*)-3-hydroxybutyrate. Enzymatic hydrolysis will also yield a dicarboxylic acid, which is in especially preferred aspects a metabolically relevant compound such as a dicarboxylic acid of the citric acid cycle. Advantageously, contemplated compounds have significant solubility in aqueous media, have desirable palatability and chemical stability, and can serve as a substrate for various stereoselective hydrolytic enzymes (*e.g*., lipases, esterases).

In one aspect of the inventive subject matter, the inventors contemplate a nutrient composition that comprises a nutritionally acceptable carrier in combination with a diester compound having a structure according to Formula I;

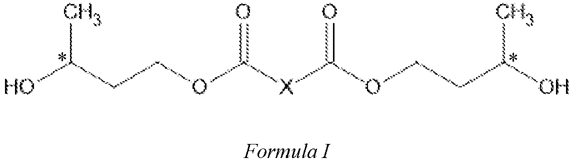

wherein X is a linear alkyl group, optionally containing at least one double bond, and optionally substituted with a substituent selected from the group consisting of a methyl group, a hydroxyl group, an amino group, and a keto group, and wherein * denotes a chiral carbon atom, and wherein the nutrient composition is formulated for oral administration.

In some embodiments, X is (CH₂)ₙ, and wherein n is an integer between 1 and 10. In further embodiments, the linear alkyl group is substituted with at least one keto group. In still further embodiments, the linear alkyl group is substituted with at least one hydroxyl group. In yet further embodiments, the linear alkyl group is substituted with at least one amino group. In further embodiments, the linear alkyl group is substituted with at least one methyl group. In still further embodiments, the linear alkyl group has at least one double bond.

It is also contemplated that at least one of the chiral carbon atoms has an (*R*)-configuration, and/or that the diester compound is a substrate for a lipase or an esterase. Therefore, it is contemplated that the diester compound, upon hydrolysis by the lipase or the esterase, produces (*R*)-1,3-butanediol and/or produces a metabolically relevant dicarboxylic acid. For example, metabolically relevant dicarboxylic acids include intermediates in a tricarboxylic acid (TCA) cycle, intermediates or substrates for beta oxidation, intermediates or substrates for glycolysis, and/or intermediates or substrates for gluconeogenesis. Thus, contemplated metabolically relevant dicarboxylic acid especially include succinic acid, malic acid, fumaric acid, and alpha-ketoglutaric acid.

Consequently, the inventors contemplate that the diester compound may have a structure according to Formula II, III, IV, or V

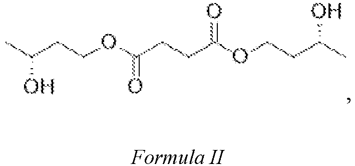

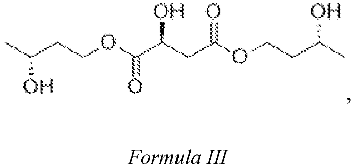

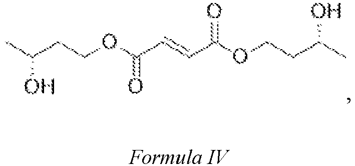

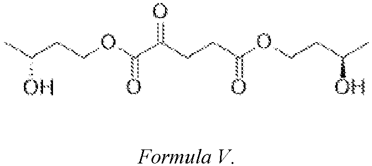

Where desired, contemplated compositions may further comprise (*R*)-1,3-butanediol, (*R*)-3-hydroxybutyrate, and/or a dicarboxylic acid. As will be appreciated, contemplated compositions may be formulated as a liquid such as a liquid concentrate, a ready-to-drink beverage, or a gel, or may be formulated as a solid, such as a bulk powder, a tablet or capsule, a lozenge, a dissolving film, or a snack bar.

In some embodiments, the composition may be formulated into a solid dosage unit to provide between 20 mg and 1,000 mg of the diester compound per dosage unit to a consumer ingesting the composition dosage unit. In other embodiments, the composition may be formulated into a liquid dosage unit to provide between 2 g and 30 g of the diester compound per dosage unit to a consumer ingesting the composition dosage unit.

Therefore, in a further aspect of the inventive subject matter, the inventors also contemplate a method of increasing (*R*)-3-hydroxybutyrate in a mammal that includes a step of orally administering to the mammal a nutrient composition as presented herein. Most typically, an enzymatic conversion of the diester compound in the mammal will then produce (*R*)-1,3-butanediol and a dicarboxylic acid, and a further enzymatic conversion of the (*R*)-1,3-butanediol in the mammal will then produce (*R*)-3-hydroxybutyrate.

For example, the composition may be administered in a solid dosage unit to provide between 20 mg and 1,000 mg or between 100 mg and 2,000 mg of the diester compound to the mammal, typically in form of one or more tablets or capsules. On the other hand, the composition may be administered in a liquid dosage unit to provide between 2 g and 30 g of the diester compound to the mammal, typically in form of a gel or ready-to-drink item (such as an energy drink or flavored beverage).

In still another aspect of the inventive subject matter, the inventors also contemplate a method of producing a nutrient composition that includes a step of combining a diester compound with a nutritionally acceptable carrier to thereby produce the nutrient composition. Most typically, the diester compound will have a structure according to Formula I;

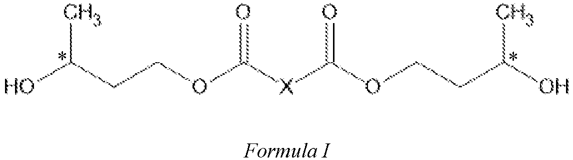

wherein X is a linear alkyl group, optionally containing at least one double bond, and optionally substituted with a substituent selected from the group consisting of a methyl group, a hydroxyl group, an amino group, and a keto group, and wherein * denotes a chiral carbon atom, and in a further step, the nutrient composition is formulated for oral administration.

In some embodiments, the diester compound may be produced by direct esterification of a metabolically relevant dicarboxylic acid and 1,3-butanediol (and especially (*R*)-1,3-butanediol)). Among other choices, the metabolically relevant dicarboxylic acid may be an intermediate in the tricarboxylic acid (TCA) cycle, an intermediate or substrate for beta oxidation, an intermediate or substrate for glycolysis, or an intermediate or substrate for gluconeogenesis. Thus, suitable metabolically relevant dicarboxylic acid include succinic acid, malic acid, fumaric acid, and alpha-ketoglutaric acid. Preferably, but not necessarily, the direct esterification comprises an enzymatic esterification.

In other embodiments, the diester compound may be produced by transesterification of a dicarboxylic acid diester intermediate and 1,3-butanediol (and especially (*R*)-1,3-butanediol)). For example, suitable dicarboxylic acid diester intermediates may be diesters of ethanol and a metabolically relevant dicarboxylic acid, wherein the metabolically relevant dicarboxylic acid may be an intermediate in the tricarboxylic acid (TCA) cycle, an intermediate or substrate for beta oxidation, an intermediate or substrate for glycolysis, or an intermediate or substrate for gluconeogenesis. Thus, suitable metabolically relevant dicarboxylic acid include succinic acid, malic acid, fumaric acid, and alpha-ketoglutaric acid. Preferably, but not necessarily, the transesterification comprises an enzymatic transesterification.

In further contemplated embodiments of such methods, X is (CH₂)ₙ, and n is an integer between 1 and 10. Therefore, suitable diester compounds may have a structure according to Formula II, III, IV, or V

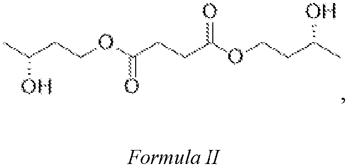

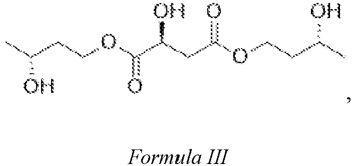

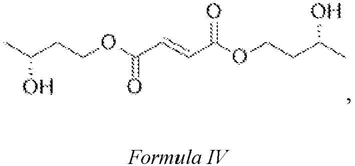

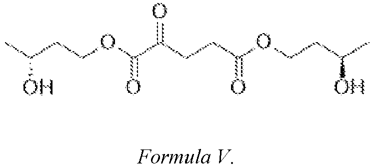

Various objects, features, aspects, and advantages of the inventive subject matter will become more apparent from the following detailed description of preferred embodiments, along with the accompanying drawing figures in which like numerals represent like components.

### Brief Description of The Drawing

**FIG. 1** is a schematic illustrating a metabolic pathway for enzymatic degradation of a first exemplary compound according to the inventive subject matter.
**FIG. 2** is a schematic illustrating a metabolic pathway for enzymatic degradation of a second exemplary compound according to the inventive subject matter.
**FIG. 3** is a schematic illustrating a metabolic pathway for enzymatic degradation of a third exemplary compound according to the inventive subject matter.
**FIG. 4** is a schematic illustrating a metabolic pathway for enzymatic degradation of a fourth exemplary compound according to the inventive subject matter.

### Detailed Description

The inventors have discovered various compounds, compositions, and methods of providing (*R*)-3-hydroxybutyrate and a metabolically relevant compound to an individual. Most preferably, contemplated compounds are prepared in a conceptually simple and efficient manner, have high solubility in aqueous media, desirable palatability, and will allow for *in vivo* selective production of (*R*)-3-hydroxybutyrate. In further advantageous aspects, it should also be appreciated that contemplated compounds will deliver for each mol of the metabolically relevant compound two moles of (*R*)-1,3-butanediol, which can then be further enzymatically oxidized to (*R*)-3-hydroxybutyrate.

In this context, it should be appreciated that among other contemplated metabolically relevant compounds, especially preferred metabolically relevant compounds include various dicarboxylic acids that are an intermediate or substrate in the tricarboxylic acid (TCA) cycle, an intermediate or substrate for beta oxidation, an intermediate or substrate for glycolysis, or an intermediate or substrate for gluconeogenesis. Therefore, exemplary preferred metabolically relevant compounds include succinic acid, malic acid, fumaric acid, and alpha-ketoglutaric acid. However, various other substituted and unsubstituted dicarboxylic acids and tricarboxylic acids are also deemed suitable for use herein and are discussed in more detail below. In addition, it should be noted that while diesters with (*R*)-1,3-butanediol are generally preferred, mixed diesters formed with (*S*)-1,3-butanediol and (*R*)-1,3-butanediol are also deemed suitable for use herein.

Therefore, and viewed from a different perspective, the inventors generally contemplate various compositions containing a compound having the structure of Formula I: 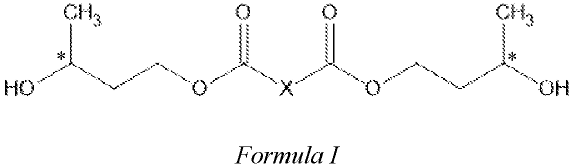 wherein X is a linear alkyl group, optionally containing at least one double bond, and optionally substituted with a substituent selected from the group consisting of a methyl group, a methylene group, a hydroxyl group, an amino group, and a keto group, and wherein * denotes a chiral carbon atom. Preferably, but not necessarily, * independently represents the (*R*)-configuration.

In most typical examples, X is (CH₂)ₙ, and n is an integer between 1 and 10, and as can be seen from Tables 1-3 below, contemplated compounds may be further substituted with a keto group, a methyl group, a methylene group, and amino group (and especially where X is (CH₂)ₙ, and wherein n is an integer greater than 3), and/or a hydroxyl group. In less typical examples, the compounds according to the inventive subject matter may also have one or more double bonds.

As will be readily appreciated, contemplated compounds are preferably the formal reaction products of one molecule of a metabolically relevant (di/tri)carboxylic acid and two molecules of (*R*)-1,3-butanediol. Thus, and viewed from a different perspective, contemplated compounds will be hydrolysable (preferably enzymatically) into one molecule of a metabolically relevant (di/tri)carboxylic acid and two molecules of (*R*)-1,3-butanediol. Among other suitable options, contemplated diester or triester compounds will typically be cleavable by a lipase or an esterase. Therefore, in most instances contemplated diester or triester compounds will generate (R)-1,3-butanediol as a reaction product along with the corresponding metabolically relevant (di/tri)carboxylic acid.

For example, suitable linear saturated dicarboxylic acids are shown in **Table 1,** while exemplary substituted dicarboxylic acids are shown in **Table 2.** Exemplary unsaturated dicarboxylic acids are shown in **Table 3,** and contemplated tricarboxylic acids are shown in

### Table 4.

**Table 1**

| Linear Saturated Dicarboxylic Acids | | |
|---|---|---|
| Trivial name | IUPAC name | Structure |
| Oxalic acid | ethanedioic acid | 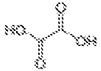 |
| Malonic acid | propanedioic acid | 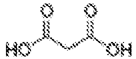 |
| Succinic acid | butanedioic acid | 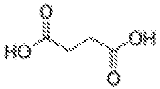 |
| Glutaric acid | pentanedioic acid | 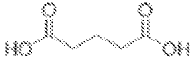 |
| Adipic acid | hexanedioic acid | 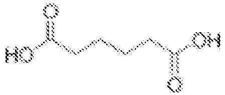 |
| Pimelic acid | heptanedioic acid | 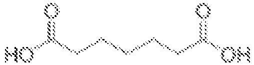 |
| Suberic acid | octanedioic acid | 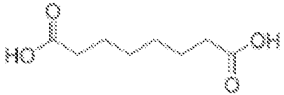 |
| Azelaic acid | nonanedioic acid | 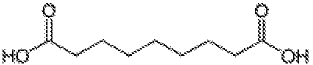 |
| Sebacic acid | decanedioic acid | 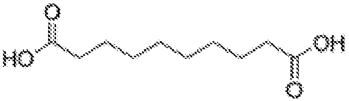 |
| | undecanedioic acid | 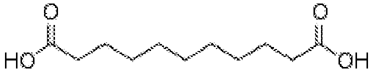 |
| | dodecanedioic acid | 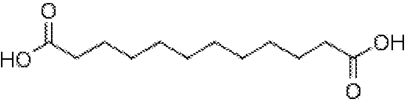 |
| Brassylic acid | tridecanedioic acid | 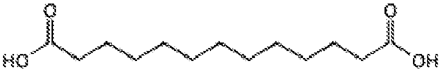 |
| Thapsic acid | hexadecanedioic acid | 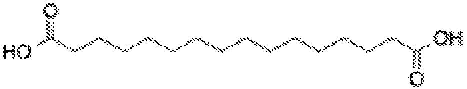 |

**Table 2**

| Substituted Dicarboxylic Acid | | |
|---|---|---|
| Trivial Name | IUPAC name | Structural formula |
| Tartronic acid | 2-Hydroxypropanedioic acid | 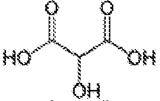 |
| Mesoxalic acid | Oxopropanedioic acid | 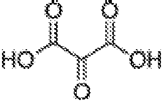 |
| Malic acid | Hydroxybutanedioic acid | 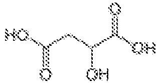 |
| Tartaric acid | 2,3-Dihydroxybutanedioic acid | 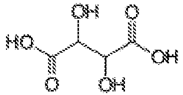 |
| Oxaloacetic acid | Oxobutanedioic acid | 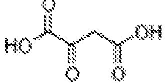 |
| Aspartic acid | 2-Aminobutanedioic acid | 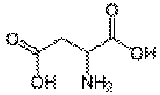 |
| dioxosuccinic acid | Dioxobutanedioic acid | 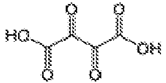 |
| α-hydroxyGlutaric acid | 2-hydroxypentanedioic acid | 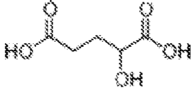 |
| Acetonedicarboxylic acid | 3-Oxopentanedioic acid | 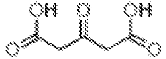 |
| α-Ketoglutaric acid | 2-Oxopentanedioic acid | 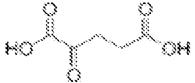 |
| Glutamic acid | 2-Aminopentanedioic acid | 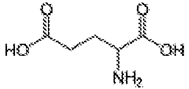 |
| Diaminopimelic acid | (2R,6S)-2,6-Diaminoheptanedioic acid | 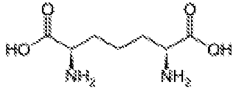 |
| Saccharic acid | (2S,3S,4S,5R)-2,3,4,5-Tetrahydroxyhexanedioic acid | 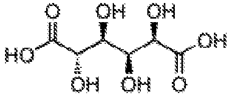 |

**Table 3**

| Unsaturated Dicarboxylic Acid | | | | |
|---|---|---|---|---|
| Type | Common name | IUPAC name | Isomer | Structural formula |
| Monounsaturated | Maleic acid | (Z)-Butenedioic acid | cis | 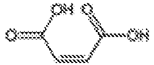 |
| | Fumaric acid | (E)-Butenedioic acid | trans | 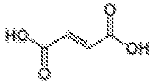 |
| | Acetylenedicarboxylic acid | But-2-ynedioic acid | not applicable | 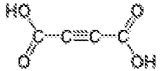 |
| | Glutaconic acid | (Z)-Pent-2-enedioic acid | cis | 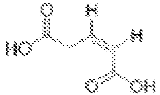 |
| | | (E)-Pent-2-enedioic acid | trans | 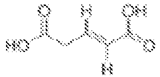 |
| | | 2-Decenedioic acid | trans | 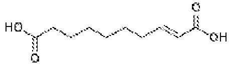 |
| | Traumatic acid | Dodec-2-enedioic acid | trans | 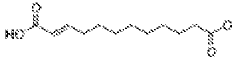 |
| Diunsaturated | Muconic acid | (2E,4E)-Hexa-2,4-dienedioic acid | trans,trans | 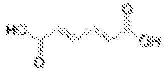 |
| | | (2Z,4E)-Hexa-2,4-dienedioic acid | cis,trans | 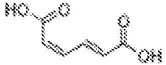 |
| | | (2Z,4Z)-Hexa-2,4-dienedioic acid | cis,cis | 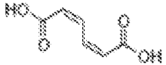 |
| | Glutinic acid (Allene-1,3-dicarboxylic acid) | (RS)-Penta-2,3-dienedioic acid | | HO₂CCH=C=CHCO₂H |
| Branched | Citraconic acid | (2Z)-2-Methylbut-2-enedioic acid | cis | 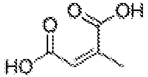 |
| | Mesaconic acid | (2E)-2-Methyl-2-butenedioic acid | trans | 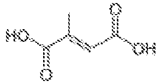 |
| | Itaconic acid | 2-Methylidenebutanedioic acid | - | 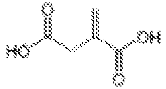 |

**Table 4**

| Trivial name | IUPAC name | Structural formula |
|---|---|---|
| Citric acid | 2-hydroxypropane-1,2,3-tricarboxylic acid | 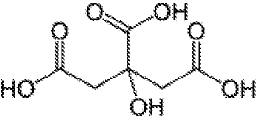 |
| Isocitric acid | 1-hydroxypropane-1,2,3-tricarboxylic acid | 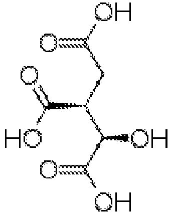 |
| Aconitic acid | Prop-1-ene-1,2,3-tricarboxylic acid | 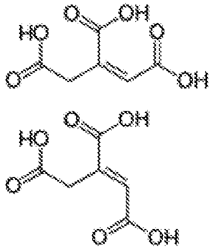 (cis-form & trans-form) |

In some embodiments, the metabolically-relevant carboxylic acid can be further characterized as a di- or tricarboxylic acid that is part of microbial or eukaryotic metabolism (anabolism and/or catabolism) in which the di- or tricarboxylic acid is an intermediate or a substrate. As such, contemplated compounds can be used to supplement an individual with both, (*R*)-1,3-butanediol (to so generate *in vivo* (*R*)-3-hydroxybutyrate) and a metabolically relevant dicarboxylic or tricarboxylic acid.

Therefore, exemplary compounds within the scope of the compound having a structure according to Formula I are compounds having a structure according to Formulae II-V:

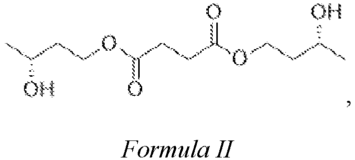

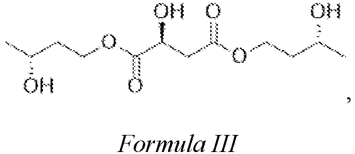

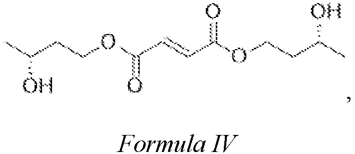

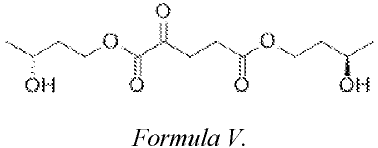

In further aspects of the inventive subject matter, it should be recognized that synthesis of contemplated compounds is conceptually simple and effective and can be performed under stereochemically controlled conditions at high yields. In general, the metabolically relevant dicarboxylic or tricarboxylic acid is reacted with 1,3-butanediol (preferably (*R*)-1,3-butanediol) to thereby form the corresponding diester or triester compound. While numerous manners of esterification are deemed suitable for use herein, particularly preferred methods include enzyme-mediated direct esterification or enzyme-mediated transesterification.

For example, the dicarboxylic acid or the tricarboxylic acid in direct esterification can be reacted with an excess of (*R*)-1,3-butanediol in the presence of a catalyst (typically an appropriate enzyme, or an organic or inorganic acid or salt). Technically, direct esterification can be done in both homogenous or heterogenous manner (*e.g*., applying immobilized enzymes or acidic resins) in batch mode or flow mode. Resulting water production from the esterification reaction may be continuously removed, for example, by adsorption/chemisorption using hygroscopic salts or molecular sieves, may be distilled off as a component of an azeotropic mixture.

Transesterification will typically require a diethyl ester of a dicarboxylic acid or tricarboxylic acid and an excess of (*R*)-1,3-butanediol and a suitable the catalyst, typically an appropriate enzyme, or an organic or inorganic acid or salt. Transesterification can be performed in both homogenous or heterogenous manner (*e.g*., applying immobilized enzymes or acidic resins) in batch mode or flow mode. In contrast to direct esterification, transesterification produces ethanol, which needs to be (constantly) removed. As ethanol is more volatile than water, ethanol can be distilled off as a component of an azeotropic mixture *or per se* under reduced pressure.

For example, suitable enzymes include commercially available lipases such as those from *Candida antartica, Candida cylinderacea, Mucor meihei, Pseudomonas cepacia, Pseudomonas fluorescens* and suitable methods for enzymatic esterification can be found in Zaccone F et al. An Alternative Enzymatic Route to the Ergogenic Ketone Body Ester (R)-3-Hydroxybutyl (R)-3-Hydroxybutyrate. Catalysts. 2021; 11(1):1. In typical exemplary embodiments, the enzyme/substrate mixture is maintained at a temperature of about 30 °C under reduced pressure for a predetermined amount of time to allow for a desired degree of enzymatic esterification. Further exemplary reactions for direct esterification and transesterification are provided in the section entitled 'Examples' below. The desired reaction product(s) can then be isolated using methods well known in the art and suitable methods include distillation, membrane filtration, adsorption to a solid phase, and various chromatographic methods.

During formation of the compound, the 1,3-butanediol may be present in excess. In certain embodiments, the 1,3-butanediol is present in excess in a molar ratio of the 1,3-butanediol to metabolically-relevant carboxylic acid of from 2:1 to 20:1, optionally from 4:1 to 10:1, or optionally about 5:1. The method may further comprise isolating excess 1,3-butanediol from the mixture. The step of isolating excess 1,3-butanediol may comprise vacuum distillation,. The excess 1,3-butanediol may be recovered (*e.g.,* 80% recovery) and repurposed for other reactions or recycled to the same reaction. Alternatively, excess or even residual 1,3-butanediol need not be removed from the reaction as 1,3-butanediol is nutritionally acceptable and may serve as a precursor for 3-hydroxybutyrate. In these and other embodiments, it should be recognized that (*R*)-1,3-butanediol is inexpensive, food friendly, and natural. More importantly, the use of (*R*)-1,3-butanediol as a precursor leads by way of hepatic enzymatic oxidation to the formation of the (*R*) configuration of 3-hydroxybutyrate, which is the metabolically relevant enantiomer of 3-hydroxybutyrate. Likewise, contemplated compounds can be enriched, isolated or purified removing undesired impurities via ion exchange or other types of chromatography. For example, unesterified or partially esterified di/tricarboxylic acids having free acidic moieties can be immobilized on ion exchange resin while desired compounds will not bind to such resins.

Upon synthesis and optional purification of the diesters or triesters contemplated herein, it should be appreciated that the compounds according to the inventive subject matter can be used as a nutrient that provides or supports or replenishes (*R*)-3-hydroxybutyrate and/or a metabolically relevant compound. Consequently, compositions comprising the compounds according to the inventive subject matter are also provided. Most preferably, such compositions will be for oral administration. However, other routes of administration, and particularly systemic administration via injection or infusion are also deemed suitable. Non-limiting examples of preferred compositions include nutritional supplements and pharmaceutical compositions. Nutritional supplements may be in a liquid or solid form comprising contemplated compounds and will typically also include a nutritionally acceptable carrier. As will be readily appreciated, the nutritionally acceptable carrier may also function to provide a specific texture or physical parameter and may assist in solidification of compounds (or adsorption to a solid phase) where the compounds are liquid at room temperature.

For example, where the nutritional supplement is in solid form, the compositions may be formulated as a snack bar, lozenge, bulk powder, dissolving film, tablet, or capsule, or may be coated onto cereal products, or included in baked goods. On the other hand, where the supplement is in liquid form, the compositions may be formulated as a ready-to-drink beverage, a liquid concentrate for admixture with an aqueous solution, a gel, a carbonated drink, a brewed beverage (*e.g*., as coffee or tea), a juice, an energy drink, a sports drink, or flavored water. In addition, pharmaceutical compositions comprising the compound may be formulated, typically as a liquid for oral administration or infusion.

Preferably, but not necessarily, contemplated compositions will be formulated for oral consumption in dosage units to assist a user in consumption of the composition in a desired quantity. For example, where the composition is formulated into a solid dosage unit, such dosage unit may be designed to provide between 20 mg and 1,000 mg of the diester compound to the consumer ingesting the composition dosage unit. On the other hand, the composition may also be formulated into a liquid dosage unit to provide larger quantities such as for example between 2 g and 30 g of the diester compound per dosage unit to a consumer ingesting the composition dosage unit.

While nutritional and pharmaceutical compositions for human use are especially contemplated, it should be appreciated that the compounds and formulations may also be employed for veterinary use (*e.g.,* use in animal feed for domestic companion animals ('pets') or in animal feed for farm animals. In further contemplated aspects, the compound may also be provided as a bulk product (*e.g.,* in quantities of equal or greater than 100 g, equal or greater than 1,000 g, or equal or greater than 10 kg) for use in production of a nutritional or pharmaceutical product.

Depending on the particular formulation, contemplated compositions may comprise at least 5 mg, more typically at least 50 mg, and most typically at least 100 mg of the compounds presented herein, depending on the serving size or dosage unit. Therefore, viewed from another perspective and depending on the particular type of final product (*e.g*., energy or flavored drink, or fortified solid food item, or capsule/tablet), contemplated compounds may be present in the composition at a concentration of between 0.0001 wt% to 0.01 wt%, or between 0.01 wt% to 0.1 wt%, or between 0.1 wt% to 1.0 wt%, or between 1.0 wt% to 10 wt%, or between 10 wt% to 99 wt%. Suitable dosages for contemplated compounds will generally be between 100 mg and 50 g, and even higher. Most commonly, dosage units are given in a single event (*e.g.,* via oral capsule, consumption of a beverage, etc.), but it is noted that the dosage units may also be given over two or more administrations.

Therefore, and viewed from another perspective, contemplated compounds may be used to deliver a metabolically relevant dicarboxylic acid or tricarboxylic acid together with (*R*)-3-hydroxybutyrate to a mammal to support or generate a ketotic state in a mammal, and/or to counteract (*e.g.*, age related) depletion of (*R*)-3-hydroxybutyrate. Notably, the presence of the metabolically relevant dicarboxylic acid or tricarboxylic acid may enhance, and in some cases synergistically enhance physiologic effects of the (*R*)-3-hydroxybutyrate. It should also be appreciated that the as compounds contemplated herein will be successively metabolized in various physiological compartments, availability and activity of the *in vivo* generated reaction products may have different effects in the different physiological compartments (*e.g*., effect on microbiome, intestinal tissues, cellular metabolism and/or energy production, etc.).

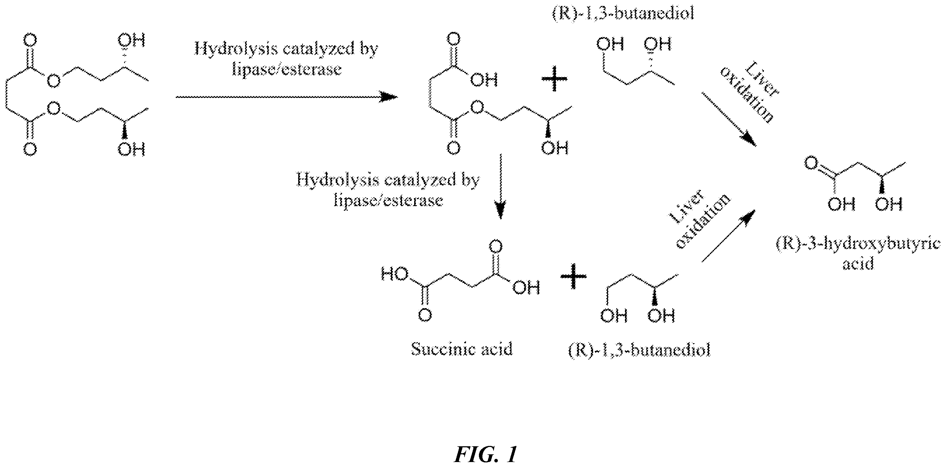

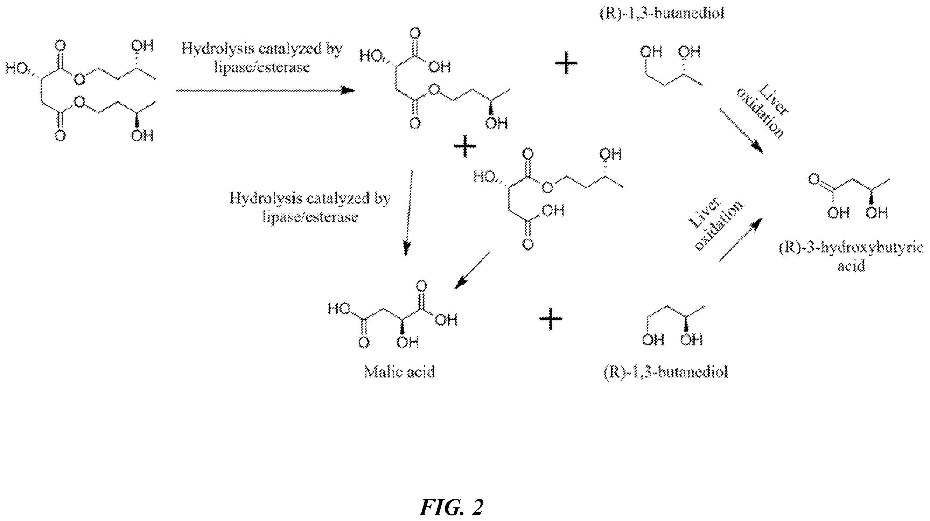

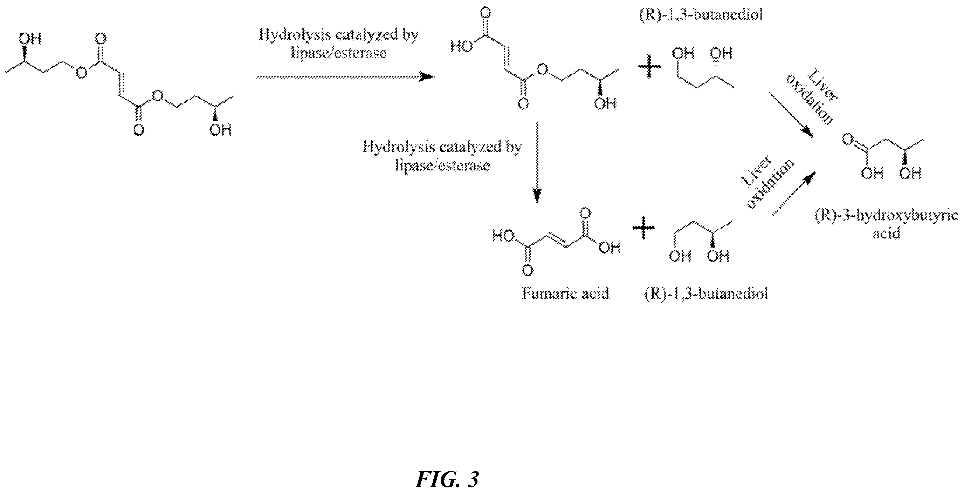

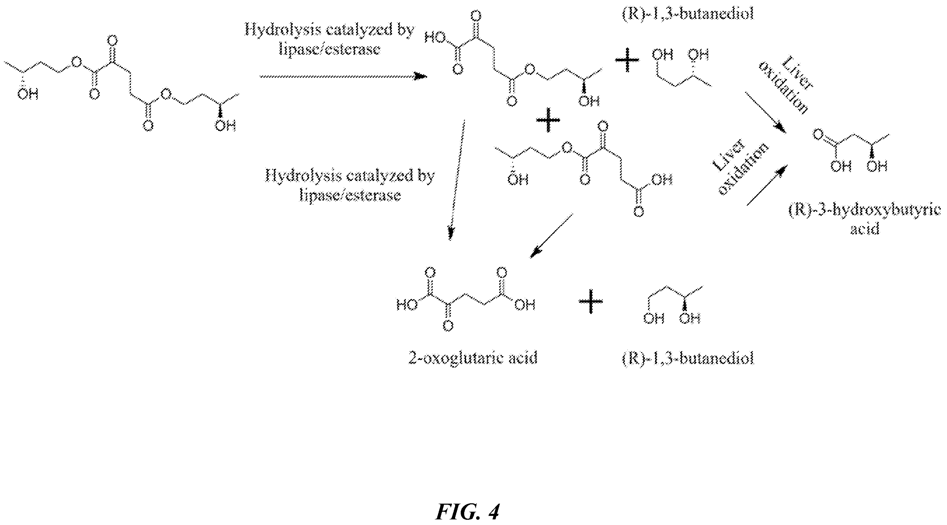

Exemplary *in vivo* conversions are schematically illustrated for selected compounds in **FIGS.1-4****.** More specifically, exemplary compound 1 in **FIG.1** may be hydrolyzed *in vivo* by an esterase found in one or more physiological compartments to form succinic acid and (*R*)-1,3-butanediol. The exemplary compound 2 in **FIG.2** may be hydrolyzed *in vivo* by an esterase found in one or more physiological compartments to form malic acid and (*R*)-1,3-butanediol, while the exemplary compound 3 in **FIG.3** may be hydrolyzed *in vivo* by an esterase found in one or more physiological compartments to form fumaric acid and (*R*)-1,3-butanediol. The exemplary compound 4 in **FIG.4** may be hydrolyzed *in vivo* by an esterase found in one or more physiological compartments to form ketoglutaric acid and (*R*)-1,3-butanediol. The resulting (*R*)-1,3-butanediol may then be metabolized in the liver to form (*R*)-3-hydroxybutyrate.

For example, lipases and/or esterases in saliva and intestinal fluid may primarily release the dicarboxylic acid or tricarboxylic acid, while (*R*)-1,3-butanediol may be metabolized in the liver to form (*R*)-3-hydroxybutyrate. As such, the different reaction products may be available at different times and different places in an organism ingesting the compounds presented herein. Notably, hydrolysis of the diester or triester compounds can lead to the formation of two or three molecules of (*R*)-1,3-butanediol for each diester or triester molecule at different points in time. As such, this initial and subsequent delayed release of (*R*)-1,3-butanediol can lead to an initial and delayed formation of (*R*)-3-hydroxybutyrate.

In various embodiments, the composition will be substantially free of carbohydrates. The phrase "substantially free" as utilized herein with regard to carbohydrates means that carbohydrates may be present in an amount of no greater than 1, optionally no greater than 0.1, optionally no greater than 0.01, or no greater than 0.001, wt.%, based on a total weight of the composition. The term "carbohydrates" as utilized herein refers to sugars, starches, and fiber that can be metabolized to glucose.

It is also contemplated that the composition may comprise additional ketone precursors or supplements to be used in combination with the compound. These additional ketone precursors or supplements may include medium chain fatty acids, mineral salts, acetoacetate, other ketone esters, and other compounds that can cause a rise in blood ketone levels.

Non-limiting examples and sources of the medium chain fatty acid, or an ester thereof, include medium chain triglyceride, include coconut oil, coconut milk powder, fractionated coconut oil, palm oil, palm kernel oil, caprylic acid, isolated medium chain fatty acids, such as isolated hexanoic acid, isolated octanoic acid, isolated decanoic acid, medium chain triglycerides either purified or in natural form such as coconut oil, and ester derivatives of the medium chain fatty acids ethoxylated triglyceride, enone triglyceride derivatives, aldehyde triglyceride derivatives, monoglyceride derivatives, diglyceride derivatives, and triglyceride derivatives, and salts of the medium chain triglycerides. Ester derivatives optionally include alkyl ester derivatives, such as methyl, ethyl, propyl, butyl, hexyl, etc. Oils may be spray dried onto solid supports such as maltodextrin to facilitate delivery in powder form. The at least one medium chain triglyceride is optionally administered at between 5 grams and 50 grams, between 10 grams and 40 grams, or between 15 grams and 30 grams. As a nonlimiting example, the medium chain triglyceride is administered at 5 grams, 6 grams, 7 grams, 8 grams, 9 grams, 10 grams, 11 grams, 12 grams, 13 grams, 14 grams, 15 grams, 17 grams, 19 grams, 20 grams, 22 grams, 24 grams, 26 grams, 28 grams, 30 grams, 32 grams, 34 grams, 36 grams, 38 grams 40 grams.

Non-limiting examples of suitable mineral salts include Na, Mg, V, K, Cr, Mn, Co, Cu, Zn, As, Mo and/or Se cations associated with an appropriate counterion such as chloride, sulfate, phosphate, or other nutritionally acceptable counterions known in the art.

In still further embodiments, contemplated compositions may further comprise other nutritional substrates such as free amino acids, amino acid metabolites, vitamins, minerals, electrolytes and metabolic optimizers such as NADH, soluble ubiquinol, tetrahydrobiopterin, alpha-ketoglutaric acid, carnitine, and/or alpha lipoic acid, nutritional co-factors, calcium beta-methyl-beta-hydroxybutyrate, arginine alpha-ketoglutarate, sodium R-alpha lipoic acid, thiamine, riboflavin, niacin, pyridoxine, ascorbic acid, citric acid, malic acid, sodium benzoate, potassium sorbate, acesulfame K, aspartame, xanthan gum, or a combination thereof. Nonlimiting examples of nutritional co-factors include R-alpha lipoic acid, acetyl-1-carnitine, ketoisocaproate, alpha-ketoglutarate, alpha-hydroxyisocaproate, creatine, branched chain amino acids (leucine, isoleucine, valine), beta-hydroxy-beta methylbutyrate (HMB), B vitamins, vitamin C, soluble ubiquinol, and carnitine.

In certain embodiments, the composition comprises an encapsulant, and the compound that is at least partially encapsulated by the encapsulant. The encapsulant may comprises cyclodextrin, nanofibers, or a combination thereof. Non-limiting examples of suitable cyclodextrin encapsulates include any a cyclic dextrin molecule that is formed by enzyme conversion of starch. Specific enzymes, e.g., various forms of cycloglycosyltransferase (CGTase), can break down helical structures that occur in starch to form specific cyclodextrin molecules having three-dimensional polyglucose rings with, e.g., 6, 7, or 8 glucose molecules. For example, α-CGTase can convert starch to α-cyclodextrin having 6 glucose units, β-CGTase can convert starch to β-cyclodextrin having 7 glucose units, and γ-CGTase can convert starch to γ-cyclodextrin having 8 glucose units. Cyclodextrins include, but are not limited to, at least one of α-cyclodextrin, β-cyclodextrin, γ-cyclodextrin, and combinations thereof. The cyclodextrin may be derivatized. Suitable derivatized cyclodextrins include, but are not limited to, hydroxyalkylated cyclodextrins, such as 2-hydroxypropyl β-cyclodextrin, 3-hydroxypropyl β-cyclodextrin, 2,3-dihydroxypropyl β-cyclodextrin, and hydroxyethyl β-cyclodextrin, and methylated cyclodextrins, such as methyl β-cyclodextrin. Non-limiting examples of suitable nanofiber encapsulants include any nanofiber web or mat that is a nonwoven randomly oriented or aligned collection of nanofibers, such as those formed from various inorganic, organic, or biological polymers.

### Example

### Step 1 - Esterification of 2-oxoglutaric acid with ethanol.

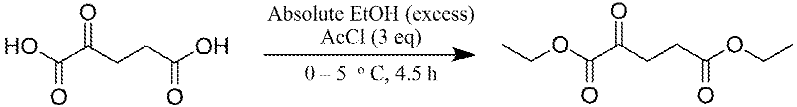

To a vigorously stirred, pre-cooled (nearly 0 ° C) solution of 2-oxoglutaric acid (30 g, 205.3 mmol) in 300 mL of absolute ethanol (> 99%) acetyl chloride (44 mL, 616 mmol, 3 eq) was added at the rate sufficient to maintain desired temperature (0 - 5 ° C, total time of adding was 1 h). The reaction was stirred at 0 - 5 ° C for additional 3.5 h. After that time, only small amount of unreacted substrate was observed in the reaction mixture (TLC monitoring: hexane/AcOEt = 7:3 v/v). Next, the mixture was diluted with 300 mL of AcOEt, washed with saturated NaHCO₃ (3x300 mL), saturated NaCl (150 mL), dried over Na₂SO₄ and filtered. The solvents were evaporated under the reduced pressure giving 38.6 g of the product with the purity 93% containing 3.2% of acetal and 3.3% of unknown impurity (GC-FID). ¹H NMR spectrum corresponded with the chemical structure.

### Step 2 - Enzymatic transesterification of diethyl 2-oxoglutarate with (R)-1,3-butanediol (batch mode).

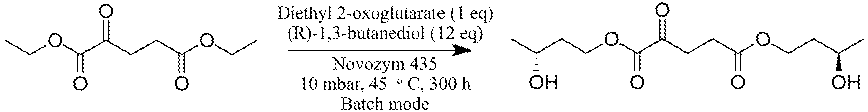

The mixture of diethyl 2-oxoglutarate (10.0 g, 49.5 mmol, 1 eq, crude intermediate from Step 1), (R)-1,3-butanediol (53.5 g, 593 mmol, 12 eq) and 2.3 g of Novozym 435 were stirred at 45 ° C and 10 mbar. The reaction progress was TLC- (CH₂Cl₂/MeOH = 9/1 v/v) and GF-FID-monitored. When reaction slowed down, additional portion of Novozym 435 was added. Totally, 3.35 g of Novozym 435 was added and total reaction time was 300 h, followed by filtering all the insoluble off. The excess of (R)-1,3-butanediol was bulb-to-bulb distilled off from the filtrate (80 ° C, 0.05 mbar, distillation time 60 min.). The resulting 10.9 g of material contained 78% of desired diester of 2-oxoglutaric acid with (R)-1,3-butanediol, 15% of R-1,3-butanediol and some unknown impurities (GC-FID). ¹H NMR spectrum corresponded with the chemical structure of the desired compound of the obtained purity.

### Step 2 - Enzymatic transesterification of diethyl 2-oxoglutarate with (R)-1,3-butanediol (flow mode).

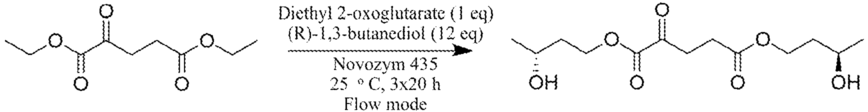

The mixture of diethyl 2-oxoglutarate (10.0 g, 49.5 mmol, 1 eq, crude intermediate from Step 1), (R)-1,3-butanediol (53.5 g, 593 mmol, 12 eq) was passed through Teflon tube Ø = 3 mm containing Novozym 435 (0.88 g) for 20 h. The product was collected into the flask, followed by evaporation the volatiles under the reduced pressure (45 ° C, 10 mbar). The procedure was repeated 3 times until complete conversion of diethyl 2-oxoglutarate was observed according to TLC (CH₂Cl₂/MeOH = 9/1 v/v) and GC-FID. The product was collected, all the volatiles were evaporated under the reduced pressure, followed by bulb-to-bulb distillation (80 ° C, 0.05 mbar, distillation time 120 min.) giving 7.45 g of final material. The resulting 7.45 g of material contained 87% of desired diester of 2-oxoglutaric acid with (R)-1,3-butanediol, 7% of R-1,3-butanediol and some unknown impurities (GC-FID). ¹H NMR spectrum corresponded with the chemical structure of the desired compound of the obtained purity.

As will be readily appreciated, numerous alternative compounds can be produced following substantially the same protocol as described in the Examples above using substituted or unsubstituted dicarboxylic acids in place of 2-oxo-glutaric acid. Suitable exemplary alternative dicarboxylic acids can be found in **Tables 1-4** above. Moreover, where the dicarboxylic acid is a substituted dicarboxylic acids containing an amino group, conventional protection groups (*e.g.*, *t*-butyloxycarbonyl, 9-fluorenylmethoxycarbonyl, etc.) can be used.

In some embodiments, the numbers expressing quantities of ingredients, properties such as concentration, reaction conditions, and so forth, used to describe and claim certain embodiments of the invention are to be understood as being modified in some instances by the term "about." Accordingly, in some embodiments, the numerical parameters set forth in the written description and attached claims are approximations that can vary depending upon the desired properties sought to be obtained by a particular embodiment. The recitation of ranges of values herein is merely intended to serve as a shorthand method of referring individually to each separate value falling within the range. Unless otherwise indicated herein, each individual value is incorporated into the specification as if it were individually recited herein.

As used herein, the term "administering" a pharmaceutical composition or drug refers to both direct and indirect administration of the pharmaceutical composition or drug, wherein direct administration of the pharmaceutical composition or drug is typically performed by a health care professional (*e.g*., physician, nurse, etc.), and wherein indirect administration includes a step of providing or making available the pharmaceutical composition or drug to the health care professional for direct administration (*e.g.*, via injection, infusion, oral delivery, topical delivery, etc.). It should further be noted that the terms "prognosing" or "predicting" a condition, a susceptibility for development of a disease, or a response to an intended treatment is meant to cover the act of predicting or the prediction (but not treatment or diagnosis of) the condition, susceptibility and/or response, including the rate of progression, improvement, and/or duration of the condition in a subject.

All methods described herein can be performed in any suitable order unless otherwise indicated herein or otherwise clearly contradicted by context. The use of any and all examples, or exemplary language (e.g., "such as") provided with respect to certain embodiments herein is intended merely to better illuminate the invention and does not pose a limitation on the scope of the invention otherwise claimed. No language in the specification should be construed as indicating any non-claimed element essential to the practice of the invention.

As used in the description herein and throughout the claims that follow, the meaning of "a," "an," and "the" includes plural reference unless the context clearly dictates otherwise. Also, as used in the description herein, the meaning of "in" includes "in" and "on" unless the context clearly dictates otherwise. As also used herein, and unless the context dictates otherwise, the term "coupled to" is intended to include both direct coupling (in which two elements that are coupled to each other contact each other) and indirect coupling (in which at least one additional element is located between the two elements). Therefore, the terms "coupled to" and "coupled with" are used synonymously.

In interpreting both the specification and the claims, all terms should be interpreted in the broadest possible manner consistent with the context. In particular, the terms "comprises" and "comprising" should be interpreted as referring to elements, components, or steps in a non-exclusive manner, indicating that the referenced elements, components, or steps may be present, or utilized, or combined with other elements, components, or steps that are not expressly referenced. Where the specification or claims refer to at least one of something selected from the group consisting of A, B, C .... and N, the text should be interpreted as requiring only one element from the group, not A plus N, or B plus N, etc.

## Claims

1. A nutrient composition, comprising:
a nutritionally acceptable carrier in combination with a diester compound having a structure according to Formula I; 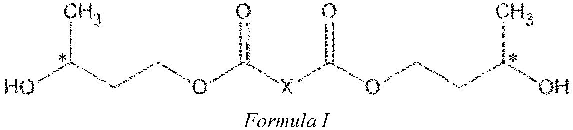
wherein X is a linear alkyl group, optionally containing at least one double bond, and optionally substituted with a substituent selected from the group consisting of a methyl group, a methylene group, a hydroxyl group, an amino group, and a keto group, and wherein * denotes a chiral carbon atom; and
wherein the nutrient composition is formulated for oral administration.

2. The composition of claim 1, wherein X is (CH₂)ₙ, and wherein n is an integer between 1 and 10, and/or wherein the linear alkyl group is substituted with at least one keto group, at least one hydroxyl group, at least one amino group, or at least one methyl group.

3. The composition of any one of the preceding claims, wherein the linear alkyl group has at least one double bond.

4. The composition of any one of the preceding claims, wherein at least one of the chiral carbon atoms has an (*R*)-configuration.

5. The composition of any one of the preceding claims, wherein the diester compound is a substrate for a lipase or an esterase, optionally wherein the diester compound upon hydrolysis by the lipase or the esterase produces (*R*)-1,3-butanediol.

6. The composition of claim 5, wherein the diester compound upon hydrolysis by the lipase or the esterase produces a metabolically relevant dicarboxylic acid, optionally wherein the metabolically relevant dicarboxylic acid is an intermediate or substrate in a tricarboxylic acid (TCA) cycle, an intermediate or substrate for beta oxidation, an intermediate or substrate for glycolysis, or an intermediate or substrate for gluconeogenesis.

7. The composition of claim 6, wherein the metabolically relevant dicarboxylic acid is selected from the group consisting of succinic acid, malic acid, fumaric acid, and alpha-ketoglutaric acid.

8. The composition of any one of the preceding claims, wherein the diester compound has a structure according to Formula II, III, IV, or V

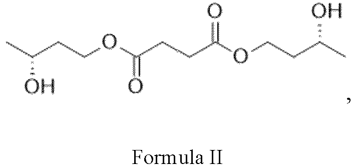

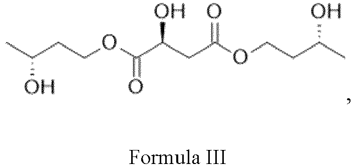

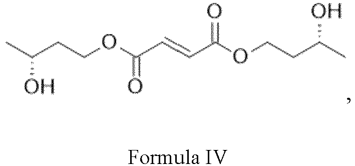

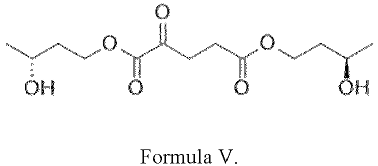

9. The composition of any one of the preceding claims, wherein the composition is formulated as a liquid concentrate, a ready-to-drink beverage, or a gel, or wherein the composition is formulated as a solid, and wherein the solid is selected from the group consisting of a bulk powder, a tablet or capsule, a lozenge, a dissolving film, or a snack bar.

10. The composition of any one of the preceding claims, wherein the composition is formulated into a solid dosage unit to provide between 20 mg and 1,000 mg of the diester compound per dosage unit to a consumer ingesting the composition dosage unit.

11. The composition of any one of the preceding claims, wherein the composition is formulated into a liquid dosage unit to provide between 2 g and 30 g of the diester compound per dosage unit to a consumer ingesting the composition dosage unit.

12. A diester compound for use in supporting or generating nutritional and/or therapeutic ketosis in a mammal, **characterized in that** the diester compound has a structure according to Formula I; 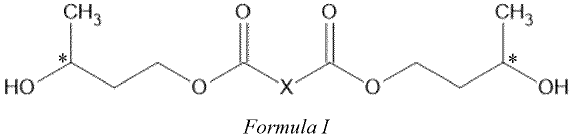
wherein X is a linear alkyl group, optionally containing at least one double bond, and optionally substituted with a substituent selected from the group consisting of a methyl group, a methylene group, a hydroxyl group, and a keto group, and wherein * denotes a chiral carbon atom; and
wherein the diester compound is formulated for oral administration.

13. The diester compound for use of claim 12, wherein the diester compound is a substrate for a lipase or an esterase, and wherein the diester compound upon hydrolysis by a lipase or an esterase produces (R)-1,3-butanediol.

14. The diester compound for use of claim 12 or claim 13, wherein the diester compound upon hydrolysis by the lipase or the esterase produces a metabolically relevant dicarboxylic acid, and wherein the metabolically relevant dicarboxylic acid is an intermediate or substrate in a tricarboxylic acid (TCA) cycle, an intermediate or substrate for beta oxidation, an intermediate or substrate for glycolysis, or an intermediate or substrate for gluconeogenesis.

15. The diester compound for use of claim 12, wherein the diester compound has a structure according to Formula II, III, IV, or V

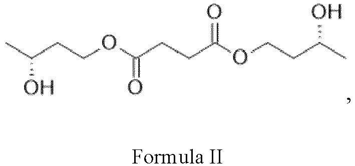

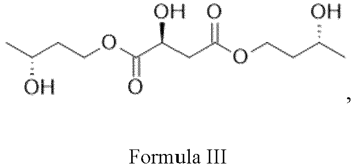

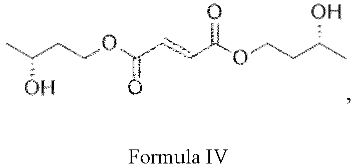

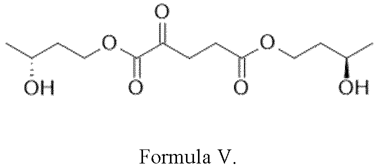

## Patentansprüche

1. Nährstoffzusammensetzung, umfassend:
einen ernährungsphysiologisch unbedenklichen Träger in Kombination mit einer Diester-Verbindung, die eine Struktur gemäß Formel I aufweist; 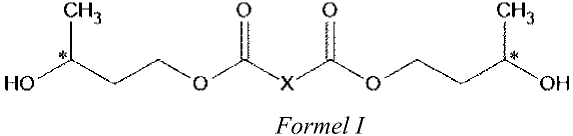
wobei X eine lineare Alkylgruppe ist, die optional mindestens eine Doppelbindung enthält und optional mit einem Substituenten, ausgewählt aus der Gruppe bestehend aus einer Methylgruppe, einer Methylengruppe, einer Hydroxygruppe, einer Aminogruppe und einer Ketogruppe, substituiert ist, und
wobei * ein chirales Kohlenstoffatom bezeichnet; und
wobei die Nährstoffzusammensetzung für die orale Verabreichung formuliert ist.

2. Zusammensetzung nach Anspruch 1, wobei X (CH₂)ₙ ist und wobei n eine ganze Zahl zwischen 1 und 10 ist und/oder wobei die lineare Alkylgruppe mit mindestens einer Ketogruppe, mindestens einer Hydroxygruppe, mindestens einer Aminogruppe oder mindestens einer Methylgruppe substituiert ist.

3. Zusammensetzung nach einem der vorhergehenden Ansprüche, wobei die lineare Alkylgruppe mindestens eine Doppelbindung aufweist.

4. Zusammensetzung nach einem der vorhergehenden Ansprüche, wobei mindestens eines der chiralen Kohlenstoffatome eine (*R*)-Konfiguration aufweist.

5. Zusammensetzung nach einem der vorhergehenden Ansprüche, wobei die Diester-Verbindung ein Substrat für eine Lipase oder eine Esterase ist, wobei optional die Diester-Verbindung bei Hydrolyse durch die Lipase oder die Esterase (*R*)-1,3-Butandiol erzeugt.

6. Zusammensetzung nach Anspruch 5, wobei die Diester-Verbindung bei Hydrolyse durch die Lipase oder die Esterase eine stoffwechselrelevante Dicarbonsäure erzeugt, wobei optional die stoffwechselrelevante Dicarbonsäure ein Zwischenprodukt oder Substrat in einem Tricarbonsäure (TCA)-Zyklus, ein Zwischenprodukt oder Substrat für die Beta-Oxidation, ein Zwischenprodukt oder Substrat für die Glykolyse oder ein Zwischenprodukt oder Substrat für die Gluconeogenese ist.

7. Zusammensetzung nach Anspruch 6, wobei die stoffwechselrelevante Dicarbonsäure ausgewählt ist aus der Gruppe bestehend aus Bernsteinsäure, Apfelsäure, Fumarsäure und Alpha-Ketoglutarsäure.

8. Zusammensetzung nach einem der vorhergehenden Ansprüche, wobei die Diester-Verbindung eine Struktur gemäß Formel II, III, IV oder V aufweist

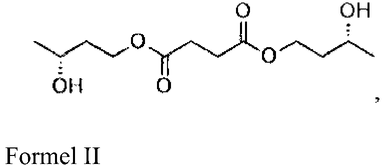

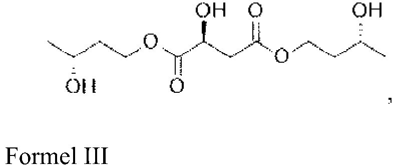

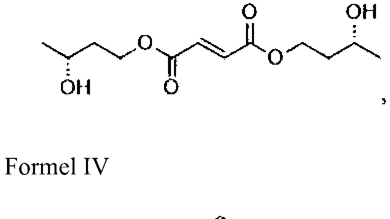

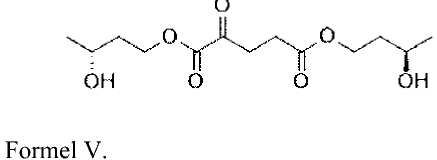

9. Zusammensetzung nach einem der vorhergehenden Ansprüche, wobei die Zusammensetzung als ein flüssiges Konzentrat, ein trinkfertiges Getränk oder ein Gel formuliert ist oder wobei die Zusammensetzung als ein Feststoff formuliert ist und wobei der Feststoff ausgewählt ist aus der Gruppe bestehend aus einem Schüttpulver, einer Tablette oder Kapsel, einer Pastille, einem sich auflösenden Film oder einem Snackriegel.

10. Zusammensetzung nach einem der vorhergehenden Ansprüche, wobei die Zusammensetzung in einer festen Dosierungseinheit formuliert ist, um zwischen 20 mg und 1.000 mg der Diester-Verbindung pro Dosierungseinheit einem Verbraucher bereitzustellen, der die Dosierungseinheit der Zusammensetzung einnimmt.

11. Zusammensetzung nach einem der vorhergehenden Ansprüche, wobei die Zusammensetzung in einer flüssigen Dosierungseinheit formuliert ist, um zwischen 2 mg und 30 mg der Diester-Verbindung pro Dosierungseinheit einem Verbraucher bereitzustellen, der die Dosierungseinheit der Zusammensetzung einnimmt.

12. Diester-Verbindung zur Verwendung bei der Unterstützung oder Generierung von ernährungsphysiologischer und/oder therapeutischer Ketose in einem Säugetier, **dadurch gekennzeichnet, dass** die Diester-Verbindung eine Struktur gemäß Formel I aufweist; 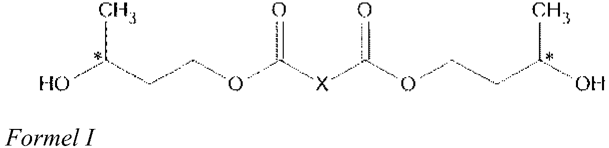
wobei X eine lineare Alkylgruppe ist, die optional mindestens eine Doppelbindung enthält und optional mit einem Substituenten substituiert ist, der ausgewählt ist aus der Gruppe bestehend aus einer Methylgruppe, einer Methylengruppe, einer Hydroxygruppe und einer Ketogruppe, und wobei * ein chirales Kohlenstoffatom bezeichnet; und
wobei die Diester-Verbindung für die orale Verabreichung formuliert ist.

13. Diester-Verbindung zur Verwendung nach Anspruch 12, wobei die Diester-Verbindung ein Substrat für eine Lipase oder eine Esterase ist und wobei die Diester-Verbindung bei Hydrolyse durch eine Lipase oder eine Esterase (R)-1,3-Butandiol erzeugt.

14. Diester-Verbindung zur Verwendung nach Anspruch 12 oder Anspruch 13, wobei die Diester-Verbindung bei Hydrolyse durch die Lipase oder die Esterase eine stoffwechselrelevante Dicarbonsäure erzeugt und wobei die stoffwechselrelevante Dicarbonsäure ein Zwischenprodukt oder Substrat in einem Tricarbonsäure (TCA)-Zyklus, ein Zwischenprodukt oder Substrat für die Beta-Oxidation, ein Zwischenprodukt oder Substrat für die Glykolyse oder ein Zwischenprodukt oder Substrat für die Gluconeogenese ist.

15. Diester-Verbindung zur Verwendung nach Anspruch 12, wobei die Diester-Verbindung eine Struktur gemäß Formel II, III, IV oder V aufweist

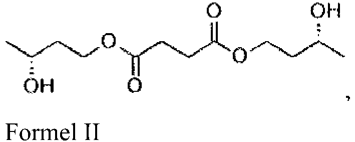

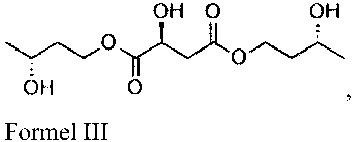

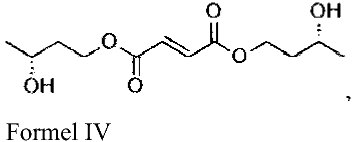

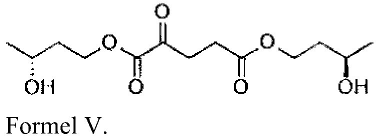

## Revendications

1. Composition nutritive comprenant :
un support nutritionnellement acceptable en combinaison avec un composé diester présentant une structure selon la Formule I ; 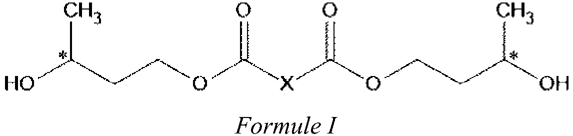
dans laquelle X est un groupe alkyle linéaire, contenant éventuellement au moins une double liaison, et
éventuellement substitué par un substituant choisi dans le groupe constitué d'un groupe méthyle, un groupe méthylène, un groupe hydroxyle, un groupe amino et un groupe céto, et
dans laquelle * désigne un atome de carbone chiral ; et
dans laquelle la composition nutritive est formulée pour une administration orale.

2. Composition de la revendication 1, dans laquelle X est (CH₂)ₙ, et dans laquelle n est un nombre entier compris entre 1 et 10, et/ou dans laquelle le groupe alkyle linéaire est substitué par au moins un groupe céto, au moins un groupe hydroxyle, au moins un groupe amino ou au moins un groupe méthyle.

3. Composition de l'une quelconque des revendications précédentes, dans laquelle le groupe alkyle linéaire a au moins une double liaison.

4. Composition de l'une quelconque des revendications précédentes, dans laquelle au moins un des atomes de carbone chiraux a une configuration (*R*).

5. Composition de l'une quelconque des revendications précédentes, dans laquelle le composé diester est un substrat pour une lipase ou une estérase, éventuellement ledit composé diester produisant, lors de l'hydrolyse par la lipase ou l'estérase, du (*R*)-1,3-butanediol.

6. Composition de la revendication 5, dans laquelle le composé diester lors de l'hydrolyse par la lipase ou l'estérase produit un acide dicarboxylique métaboliquement pertinent, ledit acide dicarboxylique métaboliquement pertinent étant éventuellement un intermédiaire ou un substrat dans un cycle d'acide tricarboxylique (TCA), un intermédiaire ou un substrat pour l'oxydation bêta, un intermédiaire ou un substrat pour la glycolyse, ou un intermédiaire ou un substrat pour la gluconéogenèse.

7. Composition de la revendication 6, dans laquelle l'acide dicarboxylique métaboliquement pertinent est choisi dans le groupe constitué de l'acide succinique, l'acide malique, l'acide fumarique et l'acide alpha-cétoglutarique.

8. Composition de l'une quelconque des revendications précédentes, dans laquelle le composé diester présente une structure selon la formule II, III, IV ou V

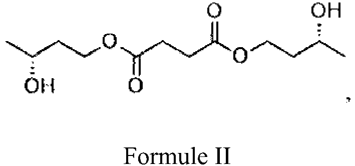

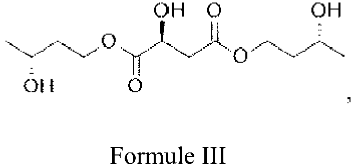

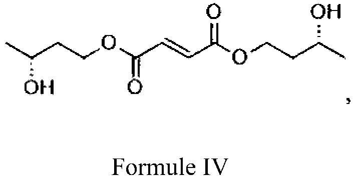

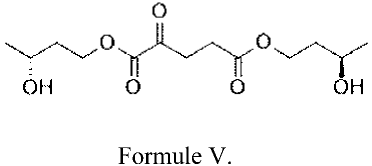

9. Composition de l'une quelconque des revendications précédentes, ladite composition étant formulée sous la forme d'un concentré liquide, d'une boisson prête à boire ou d'un gel, ou ladite composition étant formulée sous la forme d'un solide, et ledit solide étant choisi dans le groupe constitué d'une poudre en vrac, d'un comprimé ou d'une capsule, d'une pastille, d'un film dissolvant ou d'une barre de collation.

10. Composition de l'une quelconque des revendications précédentes, ladite composition étant formulée en une unité posologique solide pour fournir entre 20 mg et 1000 mg du composé diester par unité posologique à un consommateur ingérant l'unité posologique de la composition.

11. Composition de l'une quelconque des revendications précédentes, ladite composition étant formulée en une unité posologique liquide pour fournir entre 2 g et 30 g du composé diester par unité posologique à un consommateur ingérant l'unité posologique de la composition.

12. Composé diester destiné à être utilisé pour favoriser ou générer une cétose nutritionnelle et/ou thérapeutique chez un mammifère, **caractérisé en ce que** le composé diester présente une structure selon la Formule I ; 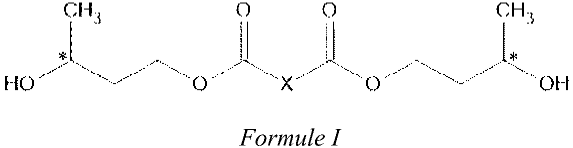
dans laquelle X est un groupe alkyle linéaire, contenant éventuellement au moins une double liaison, et
éventuellement substitué par un substituant choisi dans le groupe constitué d'un groupe méthyle, un groupe méthylène, un groupe hydroxyle et un groupe céto, et dans laquelle * désigne un atome de carbone chiral ; et
dans laquelle le composé diester est formulé pour une administration orale.

13. Composé diester destiné à être utilisé selon la revendication 12, ledit composé diester étant un substrat pour une lipase ou une estérase, et ledit composé diester produisant lors de l'hydrolyse par une lipase ou une estérase du (R)-1,3-butanediol.

14. Composé diester destiné à être utilisé selon la revendication 12 ou la revendication 13, ledit composé diester lors de l'hydrolyse par la lipase ou l'estérase produisant un acide dicarboxylique métaboliquement pertinent, ledit acide dicarboxylique métaboliquement pertinent étant un intermédiaire ou un substrat dans un cycle d'acide tricarboxylique (TCA), un intermédiaire ou un substrat pour l'oxydation bêta, un intermédiaire ou un substrat pour la glycolyse, ou un intermédiaire ou un substrat pour la gluconéogenèse.

15. Composé diester destiné à être utilisé selon la revendication 12, ledit composé diester présentant une structure selon la Formule II, III, IV ou V
